# EUROPEAN PATENT APPLICATION

(11) **EP 3 492 493 A1**
(43) Date of publication of application: **05.06.2019**
(21) Application number: 17834020.4
(22) Date of filing: 11.07.2017
(51) Int. Cl.: C07K 16/00, C07K 1/22

(54) **METHOD FOR PRODUCING ANTIBODY FRAGMENT**

(30) Priority: 28.07.2016 JP 2016148820
(71) Applicant: Kaneka Corporation, Osaka-shi, Osaka 530-8288 (JP)
(72) Inventor: MURATA, Dai, Takasago-shi Hyogo 676-8688 (JP); YOSHIDA, Shinichi, Takasago-shi Hyogo 676-8688 (JP)
(74) Representative: Hoffmann Eitle
(86) International application number: PCT/JP2017/025226
(87) International publication number: WO 2018/021012

(57) **Abstract**

The objective of the present invention is to provide a method for efficiently producing a target antibody fragment having high purity by separating an impurity which has a similar physical property to the target antibody fragment and of which separation is difficult. The method for producing an antibody fragment according to the present invention is characterized in that the antibody fragment comprises a CH1 region and does not comprise an Fc region, and characterized in comprising the steps of preparing a liquid sample, wherein the liquid sample comprises the antibody fragment and does not comprise an Fc fragment, contacting the liquid sample with an affinity separation matrix in order to adsorb the antibody fragment on the affinity separation matrix, wherein Protein G, a Protein G domain, a Protein G variant or a Protein G domain variant is immobilized as a ligand on a water-insoluble carrier in the affinity separation matrix, washing the affinity separation matrix to remove an impurity, and separating the antibody fragment from the affinity separation matrix.

## Description

### TECHNICAL FIELD

The present invention relates to a method for producing an antibody fragment which has a higher purity and which contains a CH1 region but does not contain an Fc region.

### BACKGROUND ART

As one of important functions of a protein, an ability to specifically bind to a specific molecule is exemplified. The function plays an important role in an immunoreaction and signal transduction in a living body. A technology utilizing the function for purifying a useful substance has been actively developed. As one example of proteins which are actually utilized industrially, for example, a Protein A affinity separation matrix has been used for purifying an antibody drug with high purity at one time from a culture of an animal cell (Non-patent documents 1 and 2) . Hereinafter, Protein A is abbreviated as "SpA" in some cases.

An antibody drug which has been developed is mainly a monoclonal antibody, and a monoclonal antibody has been produced on a large scale by using recombinant cell cultivation technology. A "monoclonal antibody" means an antibody obtained from a clone derived from a single antibody-producing cell. Most of antibody drugs which are presently launched are classified into an immunoglobulin G (IgG) subclass in terms of a molecular structure. Accordingly, in an initial purification step of an antibody drug production, an SpA affinity separation matrix containing SpA, which specifically binds to an Fc region of IgG, is utilized.

An antibody fragment has a molecular structure obtained by fragmenting an immunoglobulin, and various antibody fragment drugs have been clinically developed (Non-patent Document 3). Among an antibody fragment, an SpA affinity separation matrix cannot be used for purifying an antibody fragment which does not contain an Fc region. Accordingly, an affinity separation matrix capable of adsorbing an antibody fragment which does not contain an Fc region of IgG is highly required industrially in terms of a platform process for purifying an antibody drug.

A plurality of proteins which can bind to a region except for an Fc region of IgG have been already known (Non-patent Document 4). For example, as an affinity separation matrix having Protein L as a ligand, Capto L™, KappaSelect and LambdaFabSelect are known. Protein L binds to a Fab region of IgG. Hereinafter, Protein L is abbreviated as "SpL" in some cases. KappaSelect and LambdaFabSelect have a camel antibody as a ligand. The above-described affinity separation matrixes recognize only either of κ light chain or λ light chain.

The protein referred to as Protein G binds to IgG. Protein G was found from Streptococcus sp. classified in Group G. Hereinafter, Protein G is abbreviated as "SpG" in some cases. There is an SpG affinity separation matrix product (product name: "Protein G Sepharose 4 Fast Flow" manufactured by GE Healthcare, Patent Document 1) prepared by immobilizing SpG as a ligand. SpG strongly binds to an Fc region of IgG and it is also known that SpG weakly binds to a Fab region (Non-patent Documents 4 and 5). It is reported in Patent document 2 that F(ab')₂ obtained by cleaving IgG with protease is purified by an SpG affinity separation matrix on the basis this property.

### PRIOR ART DOCUMENT

### PATENT DOCUMENT

Patent Document 1: JP S63-503032 T
Patent Document 2: JP H4-49300 A

### NON-PATENT DOCUMENT

Non-patent Document 1: Hober S., et al., J. Chromatogr. B, 2007, vol. 848, pp. 40-47
Non-patent Document 2: Shukla A. A., et al., Trends Biotechnol., 2010, vol. 28, pp. 253-261
Non-patent Document 3: Nelson A. N., et al., Nat. Biotechnol., 2009, vol. 27, pp. 331-337
Non-patent Document 4: Bouvet P. J., Int. J. Immunopharmac., 1994, vol. 16, pp. 419-424
Non-patent Document 5: Derrick J. P., Nature, 1992, vol. 359, pp. 752-754
Non-patent Document 6: Andre F., et al., frontiers in IMMUNOLOGY, 2013, vol. 217, pp. 1-20
Non-patent Document 7: KOBAYASHI Kazuo, et al., Seibutsu-kougaku Kaishi, 2008, vol. 86, pp. 390-392

### DISCLOSURE OF THE INVENTION

### PROBLEMS TO BE SOLVED BY THE INVENTION

As described above, IgG is generally purified by using an affinity separation matrix containing SpA, and a ligand and an elution condition have been sufficiently studied. On the one hand, with respect to an antibody fragment, though there are a plurality of affinity separation matrixes used for purifying an antibody fragment, the affinity separation matrixes respectively have a problem and it is hard to say that sufficient study has been made.

For example, it is known to produce an antibody fragment by cleaving IgG with a protease to fragment IgG and purifying a target antibody fragment as described above. This method, however, is inefficient, since it is needed to purify a target antibody fragment from a mixture of a Fab fragment and an Fc fragment, a mixture of F(ab')₂ fragment and a further cleaved Fc fragment, or the like. Accordingly, it has been considered to selectively produce a target antibody fragment by a genetic engineering technology. Even in this method by a genetic engineering technology, a misfolded compound and unwanted component derived from an antibody are excessively produced all together in some cases in addition to a target antibody fragment (Non-patent Documents 6 and 7).

It can be thought that, for example, a Fab fragment is efficiently produced by selectively producing the Fab fragment and using an affinity separation matrix having SpL, which is a protein binding to a light chain, as a ligand with the above-described latter method. On the one hand, the present inventors found by an experiment that when a Fab fragment is produced by a genetic engineering technology, an impurity such as a light chain monomer and a light chain dimer is produced and immixed into the target Fab fragment, since SpL also adsorbs such an impurity. As described above, a genetic engineering technology has a problem that it is difficult to purify a target antibody fragment, since an impurity having a similar physical property to the target antibody fragment is produced.

Under the above-described circumstances, the objective of the present invention is to provide a method for efficiently producing a target antibody fragment having high purity by separating an impurity which has a similar physical property to the target antibody fragment and of which separation is difficult.

### MEANS FOR SOLVING THE PROBLEMS

The inventors of the present invention intensively studied for solving the above problem. As a result, the present inventors found that SpG has a specific affinity for an antibody fragment which contains a CH1 region and which does not contain an Fc region but has a low affinity for an impurity which contains a part of the target antibody fragment as a constituent element, such as a light chain monomer and a light chain dimer. Accordingly, the present inventors completed the present invention by finding that when an antibody fragment which contains a CH1 region and which does not contain an Fc region is purified from a sample which does not contain an Fc fragment, an impurity of which separation is difficult by a conventional method can be effectively separated and the target antibody fragment can be recovered with higher purity by using an affinity separation matrix having SpG as a ligand.

Hereinafter, the present invention is described.
[1] A method for producing an antibody fragment,
   wherein the antibody fragment comprises a CH1 region and does not comprise an Fc region,
   comprising the steps of:
   preparing a liquid sample, wherein the liquid sample comprises the antibody fragment and does not comprise an Fc fragment,
   contacting the liquid sample with an affinity separation matrix in order to adsorb the antibody fragment on the affinity separation matrix, wherein Protein G, a Protein G domain, a Protein G variant or a Protein G domain variant is immobilized as a ligand on a water-insoluble carrier in the affinity separation matrix,
   washing the affinity separation matrix to remove an impurity, and
   separating the antibody fragment from the affinity separation matrix.
[2] The method according to the above [1], wherein an association constant of the Protein G variant or the Protein G domain variant to the CH1 region is 10⁶ M⁻¹ or more.
[3]. The method according to the above [2], wherein the Protein G variant or the Protein G domain variant has an amino acid sequence of SEQ ID NO: 5.
[4] The method according to any one of the above [1] to [3], wherein the antibody fragment comprises a light chain, and wherein the impurity is 1 or more selected from the group consisting of a light chain monomer, a light chain dimer and an antibody aggregate.
[5] The method according to any one of the above [1] to [4], wherein an amount of a washing liquid used for washing the affinity separation matrix is 3 column volume or more.
[6] The method according to any one of the above [1] to [5], wherein an eluate used for separating the antibody fragment from the affinity separation matrix is an aqueous solution of 1 or more acids selected from the group consisting of acetic acid, citric acid and glycine.
[7] The method according to the above [6], wherein a pH of the aqueous solution is 2.5 or more and 4.0 or less.

### EFFECT OF THE INVENTION

According to the present invention method, an impurity of which physical property is similar to a target antibody fragment, such as a misfolded compound, can be removed and the target antibody fragment can be purified with higher purity after a method for selectively producing the target antibody fragment more efficiently by using a genetic engineering technology in comparison with a method for fragmenting an antibody and further purifying the target antibody fragment. Thus, according to the present invention method, it becomes possible to reduce the load of examining the condition in the latter purification stage and it becomes easy to build a purification process, since an impurity of which physical property is similar to a target antibody fragment is not brought into the latter purification stage.

### BRIEF DESCRIPTION OF THE DRAWINGS

Figure 1 is a chromatogram in the case where a CH1 region-containing Fab fragment in a culture supernatant of a yeast transfectant was purified by using a Protein G variant carrier prepared by immobilizing a Protein G variant into which mutations were introduced.
Figure 2 is a chromatogram in the case where a CH1 region-containing Fab fragment in a culture supernatant of a yeast transfectant was purified by using a commercially available Protein G affinity separation matrix prepared by immobilizing a wild Protein G.
Figure 3 is a chromatogram in the case where a CH1 region-containing Fab fragment in a culture supernatant of a yeast transfectant was purified by using a commercially available Protein L affinity separation matrix prepared by immobilizing Protein L.
Figure 4 is the SDS-PAGE result of each fraction in Figure 1 and Figure 2 in a reductive condition and a non-reductive condition.
Figure 5 is an expanded figure around 45 to 66 kDa of the SDS-PAGE gel of Figure 4 in a non-reductive condition.
Figure 6 is the SDS-PAGE result of each fraction in Figure 3 in a reductive condition and a non-reductive condition.

### MODE FOR CARRYING OUT THE INVENTION

In the present invention method, an antibody fragment which contains a CH1 region and which does not contain an Fc region is efficiently purified with high purity by using an affinity separation matrix on which Protein G, a Protein G domain, a Protein G variant or a Protein G domain variant is immobilized. Hereinafter, each step of the present invention method is described.

### Step 1: Step of preparing crude antibody fragment sample

In Step 1, a liquid sample which contains an antibody fragment containing a CH1 region and not containing an Fc region and which does not contain an Fc fragment is prepared. A method for preparing such a liquid sample is not particularly restricted as long as a target antibody fragment can be selectively produced and an impurity containing an Fc region is not produced as a by-product by the method. For example, the liquid sample can be prepared by a genetic engineering technology. The term "genetic engineering technology" in the present invention means that a gene encoding a target antibody fragment is introduced into a cell to obtain a transformant and the transformant is cultivated to selectively produce the target antibody fragment. In addition, the liquid sample can be also produced by a cell-free protein synthesis system using a gene encoding a target antibody fragment.

An "immunoglobulin (Ig)" is a glycoprotein produced by a B cell of a lymphocyte and has a function to recognize a specific molecule such as a protein to be bound. An immunoglobulin has not only a function to specifically bind to a specific molecule referred to as antigen but also a function to detoxify and remove an antigen-containing factor in cooperation with other biological molecule or cell. An immunoglobulin is generally referred to as an "antibody", and the name is inspired by such functions.

All of immunoglobulins basically have the same molecular structure. The basic structure of an immunoglobulin is a Y-shaped four-chain structure. The four-chain structure is composed of two light chains and two heavy chains of polypeptide chains. A light chain (L chain) is classified into two types of λ chain and κ chain, and all of immunoglobulins have either of the chains . A heavy chain (H chain) is classified into five types of γ chain, µ chain, α chain, δ chain and ε chain, and an immunoglobulin is classified into isotypes depending on the kind of a heavy chain. An immunoglobulin G (IgG) is a monomer immunoglobulin, is composed of two γ chains and two light chains, and has two antigen-binding sites.

A lower half vertical part in the "Y" shape of an immunoglobulin is referred to as an "Fc region", and an upper half "V" shaped part is referred to as a "Fab region". An Fc region has an effector function to initiate a reaction after an antibody binds to an antigen, and a Fab region has a function to bind to an antigen. A Fab region of a heavy chain and an Fc region are bound to each other through a hinge part. Papain, which is a proteolytic enzyme and which is contained in papaya, decomposes a hinge part to cut into two Fab regions and one Fc region. The domain part close to the tip of the "Y" shape in a Fab region is referred to as a "variable region (V region) ", since there are various changes of the amino acid sequence in order to bind to various antigens. A variable region of a light chain is referred to as a "VL region", and a variable region of a heavy chain is referred to as a "VH region". A Fab region except for a V region and an Fc region are referred to as a "constant region (C region)", since there is relatively less change. A constant region of a light chain is referred to as a "CL region", and a constant region of a heavy chain is referred to as a "CH region". A CH region is further classified into three regions of CH1 to CH3. A Fab region of a heavy chain is composed of a VH region and a CH1 region, and an Fc region of a heavy chain is composed of a CH2 region and a CH3 region. There is a hinge part between a CH1 region and a CH2 region. More specifically, it is known that the binding of Protein G to a Fab region corresponds to the binding of IgG to a CH1 region (CH1γ) and a CL region (Non-patent Document 5), and the present inventors experimentally found that when a target antibody fragment containing a CH1 region is purified by Protein G, an impurity which does not contain a CH1 region and which contains a CL region can be removed.

When an antibody is cleaved by papain, two Fab fragments and one Fc fragment are obtained. When an antibody is cleaved by pepsin, one F(ab')₂ and Fc fragments cut apart are obtained. Thus, it is not comparatively efficient to purify an antibody fragment which contains a CH1 region and which does not contain an Fc region from the above-described mixture. On the one hand, a genetic engineering technology and a cell-free protein synthesis system are comparatively efficient.

In the present invention, a liquid sample which contains an antibody fragment containing a CH1 region and not containing an Fc region and which does not an Fc fragment is prepared. The antibody fragment is not particularly restricted as long as the antibody fragment contains a CH1 region and does not contain an Fc region and may be a Fab fragment prepared by fragmenting an immunoglobulin G to a Fab region only, a F(ab')₂ fragment, a Fab₂ fragment, a Fab₃ fragment, a fragment covalently bound by a drug, and a complex prepared by fusing the fragments with a recombination protein.

In Step 1, a gene encoding a target antibody fragment may be prepared by an ordinary method. For example, a gene encoding a target antibody fragment is chemically synthesized and amplified by PCR, or DNA containing a gene encoding a target antibody fragment is used as a template and the gene encoding the target antibody fragment is amplified by PCR using primers capable of amplifying the gene. Since the antibody fragment to be produced in the present invention method contains a CH1 region and does not contain an Fc region, a gene encoding an Fc region is not used. In the "Fc region" in the present invention, a part of an Fc region, such as a CH2 region and a CH3 region, is also included.

Next, a gene encoding a target antibody fragment is inserted into a vector. A vector to be inserted by the gene is not particularly restricted as long as the vector is capable of autonomous replication in a host, and plasmid DNA and phage DNA can be used as the vector. It is preferred that the vector contains a promoter which can function in a host. As the host, a fungus such as yeast; a bacterium such as Escherichia coli and Bacillus subtilis; an animal cell such as Chinese hamster ovary (CHO) cell, BHK cell, COS cell and human-derived cell; an insect cell can be used. A host into which the above-described gene is inserted is cultivated to produce the gene. After cultivation, the used host cell is removed by filtration, centrifugation or the like to obtain the liquid sample containing a target antibody fragment. Since a gene encoding an Fc region is not used in Step 1, an Fc fragment is not contained in the liquid sample.

The pH of the liquid sample is preferably neutral as about 6 or more and about 8 or less. A solvent of the liquid sample may be water only or a buffer solution having pH of about 6 or more and about 8 or less and may contain a water-miscible organic solvent such as C₁₋₄ alcohol as long as a main component of the solvent is water.

### Step 2: Adsorption step

In Step 2, the liquid sample is contacted with an affinity separation matrix prepared by immobilizing Protein G, a Protein G domain, a Protein G variant or a Protein G domain variant on an insoluble carrier as a ligand in order to adsorb the antibody fragment on the affinity separation matrix.

The affinity separation matrix used in Step 2 is prepared by immobilizing Protein G, a Protein G domain, a Protein G variant or a Protein G domain variant on an insoluble carrier as a ligand. Hereinafter, the Protein G, Protein G domain, Protein G variant or Protein G domain variant is collectively referred to as "Protein G ligand" in some cases.

Protein G (SpG) is a protein derived from a cell wall of Streptococcus sp. classified in Group G. Protein G has an ability to bind to an immunoglobulin G (IgG) of most mammals, and it is known that SpG strongly binds to an Fc region of IgG. SpG also weakly binds to a Fab region of IgG, particularly a CH1 region and a CL region.

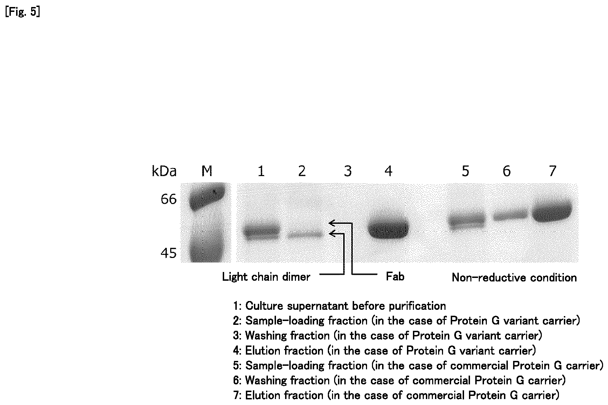

An SpG functional domain having an IgG-binding ability is referred to as "β domain", i.e. "SpG-β". The domain is referred to as β (B) domain or C domain (refer to Akerstrom et al., J. Biol. Chem., 1987, 28, p.13388-, Fig.5), but is referred to as "β domain" in this disclosure in accordance with the definition described in Fahnestock et al. (Fahnestock et al., J. Bacteriol., 1986, 167, p.870-). The details of the amino acid sequence of SpG-β are different depending on the kind and strain of a bacterium from which the SpG-β is derived. As the typical amino acid sequences, with respect to two β domains (β1 and β2) derived from Group G Streptococcus sp. GX7809 strain, the amino acid sequence of β1 domain (SpG-β1) is shown as SEQ ID NO: 2 and the amino acid sequence of β2 domain (SpG-β2) is shown as SEQ ID NO: 2. Each β domain of SpG is collectively referred to as Protein G-β domain (SpG-β), since the amino acid sequences of each β domain of SpG have high sequence identity with each other.

The term "domain" means a unit of higher-order structure of a protein. For example, a domain is composed of from dozens to hundreds of amino acid residues, and means a protein unit which can sufficiently serve some kind of a physicochemical or biochemical function.

The term "variant" of a protein or peptide means a protein or peptide obtained by introducing at least one substitution, addition and/or deletion into an amino acid sequence of a wild protein or peptide. The affinity of the "variant" in the present invention for a CH1 region is at least maintained or improved in comparison with wild SpG or a domain thereof.

Protein G (SpG) is a protein which contains 2 or 3 IgG-binding domains in the form of tandem line. As one of the embodiments, the Protein G ligand used as a ligand of the affinity separation matrix according to the present invention may be a multimer of 2 or more IgG-binding domains of wild SpG and/or SpG variant as monomers or single domains. The number of the monomers or single domains is preferably 3 or more, more preferably 4 or more, and even more preferably 5 or more. The upper limit of the number of connected domains may be, for example, 10, preferably 8, and more preferably 6. Such a multimer may be a homomultimer in which one kind of IgG-binding domains of wild SpG and/or SpG variant are connected, such as homodimer and homotrimer, or a heteromultimer in which two or more kinds of IgG-binding domains of wild SpG and/or SpG variant are connected, such as heterodimer and heterotrimer. As described above, 1 or more amino acids may be also added to the multimer composed of a plurality of domains. The position to be added is preferably an N-terminal and a C-terminal. One of the embodiments, Cys may be added to a C-terminal of 2 domain type of the IgG-binding domain of wild SpG and/or SpG variant.

It is preferred that an association constant of an SpG variant or an SpG domain variant to a CH1 region is 10⁶ M⁻¹ or more. The association constant of the Protein G ligand as a ligand of the affinity separation matrix according to the present invention to a CH1 region can be evaluated by a biosensor such as Biacore system (manufactured by GE Healthcare Bioscience) utilizing surface plasmon resonance and Octet system (manufactured by Pall) utilizing biolayer interferometry, but the means is not restricted thereto. As an association constant parameter to evaluate an affinity for a CH1 region, for example, an association constant (K_{A}) and a dissociation constant (K_{D}) can be used (Nagata et al., "Real-Time Analysis Experimental Method for Interaction Between Biological Substances", Springer-Verlag Tokyo, 1998, p. 41). A material used for evaluating an association constant of the Protein G ligand to a CH1 region is exemplified by a CH1 region peptide prepared by a genetic engineering technology; however, the CH1 region prepared by this method may not have a right structure. As another means, an association constant to 2 kinds of Fab having different antigen-recognition sites may be considered to be a quasi-association constant to a CH1 region. For example, 2 kinds of IgG of which amino acid sequences are known, of which antigen-recognition sites are different from each other but of which CH1 region homology is high are respectively fragmented by an enzyme and purified to be Fab fragments to be used. If association constants to 2 kind of Fab fragments are nearly equal, the measured association constant can be considered to be a quasi-association constant to a CH1 region.

The upper limit of an association constant to a CH1 region is not particularly restricted. The higher association constant is preferred, since an antibody fragment as a target molecule which contains a CH1 region and which does not contain an Fc region can be strongly adsorbed. On the one hand, when the association constant is excessively high, a low pH solution is needed for dissociating an adsorbed target antibody fragment; as a result, the target antibody fragment may be damaged. Accordingly, the above-described association constant is preferably, for example, 10¹¹ M⁻¹ or less.

In the experiment using Biacore system or Octet system, the order of the parameter may be largely changed depending on an experimental condition, analysis method and/or the kind of the original antibody fragment. As one of judgmental standards in such a case, it is exemplified whether a binding constant is larger or not when a peptide such as wild Protein G is evaluated in the same experimental condition and by the same analysis method. Wild Protein G is easily available as a commercially available research reagent manufactured by, for example, Life Technology.

As an SpG domain variant, the variant having the amino acid sequence of SEQ ID NO: 5 is exemplified. The SpG domain variant having the amino acid sequence of SEQ ID NO: 5 has an especially high affinity for a Fab fragment and can purify an antibody fragment containing a CH1 region and not containing an Fc region more efficiently.

A method for connecting monomer proteins used as a ligand of the affinity separation matrix according to the present invention is exemplified by a connecting method through one or more amino acid residues but is not restricted thereto. The number of the amino acid residue for connection is not particularly restricted and is preferably 20 residues or less, and more preferably 15 residues or less. It is preferred to use a sequence which connects β1 and β2 or β2 and β3 of wild SpG. From another point of view, it is preferred that the amino acid residue for connection does not destabilize a three dimensional structure of the monomer protein.

As one of the embodiments, the ligand of the affinity separation matrix according to the present invention may be a fusogenic peptide containing an IgG-binding domain of wild SpG and/or a SpG variant or a peptide multimer composed of the 2 or more domains connected each other as one constituent and other peptide having a different function. Such a fusogenic peptide is exemplified by a peptide fused with albumin or GST, i.e. glutathione S-transferase, but is not restricted to the examples. In addition, peptides fused with a nucleic acid such as DNA aptamer, a drug such as antibiotic or a polymer such as PEG, i.e. polyethylene glycol, are also included in the range of the present invention as long as such a fusogenic peptide is useful for the affinity separation matrix of the present invention.

The Protein G ligand usable in the present invention can be prepared by an ordinary method. Specifically, the DNA encoding the amino acid sequence of the desired Protein G ligand or a fragment thereof is chemically synthesized and amplified by PCR, and the amplified DNA is inserted in a vector. Escherichia coli or the like is infected with the obtained vector and cultivated, and the desired Protein G ligand may be purified from the cultivated bacterial cell or culture medium by chromatography or the like.

The affinity separation matrix used in the present invention is prepared by immobilizing the Protein G ligand on an insoluble carrier. The "insoluble carrier" usable in the present invention is insoluble in an aqueous solvent which is a solvent of the liquid sample containing an antibody fragment which contains a CH1 region and which does not contain an Fc region, and the insoluble carrier on which the ligand is immobilized can be used for purifying the above-described antibody fragment which specifically binds to the ligand. The insoluble carrier usable in the present invention is exemplified by an inorganic carrier such as glass beads and silica gel; an organic carrier; and a composite carrier obtained from the combination of the above carriers, such as an organic-organic composite carrier and an organic-inorganic composite carrier. An organic carrier is exemplified by a carrier composed of a synthetic polymer such as cross-linked polyvinyl alcohol, cross-linked polyacrylate, cross-linked polyacrylamide and cross-linked polystyrene; and a polysaccharide such as crystalline cellulose, cross-linked cellulose, cross-linked agarose and cross-linked dextran. The commercial product thereof is exemplified by porous cellulose gel GCL2000, Sephacryl S-1000 prepared by crosslinking allyl dextran and methylene bisacrylamide through a covalent bond, an acrylate carrier Toyopearl, a cross-linked agarose carrier Sepharose CL4B, and a cross-linked cellulose carrier Cellufine. However, it should be noted that the insoluble carrier usable in the present invention is not restricted to the carriers exemplified as the above.

It is preferred that the insoluble carrier usable in the present invention has large surface area and that the carrier is porous with a large number of fine pores having a suitable size in terms of a purpose of and method for using the affinity separation matrix of the present invention. The carrier can have any form such as beads, monolith, fiber, and film including hollow fiber, and any form can be selected.

As a method for immobilizing the Protein G ligand as the ligand in the present invention on an insoluble carrier, an ordinary method may be applied. For example, the ligand is immobilized by using a reactive group on the surface of an insoluble carrier. Specifically, there is a reactive group such as an amino group, a hydroxy group and a carboxy group on the surface of a general insoluble carrier. The reactive group may be activated or substituted by other reactive group, or a linker group having a reactive group may be introduced on the reactive group. For example, when an epoxy group is introduced on the surface of an insoluble carrier by using epichlorohydrin, diglycidyl ether, 1,4-bis(2,3-epoxypropoxy)butane or the like, or when an iodoacetyl group, a bromoacetyl group, a maleimide group, an N-hydroxysuccinimide group or the like is introduced on the surface of an insoluble carrier, a coupling reaction between the Protein G ligand and the reactive group can be easily accelerated.

When a linker group is used for immobilizing the ligand on an insoluble carrier, the linker group is not particularly restricted. The linker group is exemplified by a C₁₋₆ alkylene group, an amino group (-NH-), an imino group (>C=N- or -N=C<), an ether group (-O-), a thioether group (-S-), a carbonyl group (-C(=O)-), a thionyl group (-C(=S)-), an ester group (-C(=O)-O- or -O-C(=O)-), an amide group (-C(=O)-NH- or -NH-C(=O)-), a sulfoxide group (-S(=O)-), a sulfonyl group (-S(=O)₂-), a sulfonylamide group (-NH-S(=O)₂- and -S(=O)₂-NH-), and a group formed by binding a plurality of the above-described groups . When the linker group is formed by binding a plurality of the above-described groups, the number of the bound groups is preferably not more than 10 or not more than 5, and more preferably 3 or less.

A spacer molecule composed of a plurality of atoms may be introduced between the ligand and carrier. Alternatively, the ligand may be directly immobilized on the carrier. In addition, the Protein G ligand of the present invention may be chemically modified for immobilization.

In Step 2, the above-described liquid sample is contacted with the above-described affinity separation matrix in order to selectively bind the antibody fragment on the Protein G ligand in the affinity separation matrix. The specific condition for the contact is not particularly restricted, and the above-described liquid sample and the above-described affinity separation matrix may be simply mixed. Alternatively, for example, in terms of convenience, it is preferred that a column is filled with the affinity separation matrix of the present invention to be an affinity column and the liquid sample is flown through the affinity column to selectively adsorb the above-described antibody fragment on the Protein G ligand.

The condition of Step 2 may be appropriately adjusted as long as the above-described antibody fragment contained in the above-described liquid sample is sufficiently adsorbed on the above-described affinity separation matrix. For example, the pH in Step 2 may be adjusted to 6 or more and 8 or less.

### Step 3: Step of washing affinity separation matrix

In Step 3, the affinity separation matrix on which the antibody fragment is adsorbed in the above-described Step 2 is washed to remove an impurity except for the antibody fragment. Even after Step 3, the antibody fragment is adsorbed on the affinity separation matrix. On the one hand, even if an impurity such as a light chain monomer is once adsorbed, the impurity can be removed by Step 3.

As a washing liquid usable for washing the affinity separation matrix in Step 3, a washing liquid which does not disturb the interaction between the antibody fragment and the Protein G ligand is used. For example, water and a buffer of which pH is 5 or more and 8 or less can be used as the washing liquid. A usage amount of the washing liquid is represented in column volume (CV). A column volume is based on an amount of the affinity separation matrix with which a column is filled. For example, when a column is filled with 1 mL-gel of the affinity separation matrix, a usage amount of 1 CV corresponds to 1 mL. A volume of the affinity separation matrix as a standard means a volume of the affinity separation matrix in a gel condition determined by tapping or leaving still the affinity separation matrix in a suspension condition until the volume thereof is not decreased any more. An amount of the washing liquid may be appropriately determined so that an impurity can be sufficiently removed from the affinity separation matrix and is preferably 3 CV or more, more preferably 4 CV or more, and even more preferably 5 CV or more. When a usage amount of the washing liquid is larger, a removal efficiency of an impurity may become higher and a purity of the antibody fragment in the next step tends to be higher but a recovery yield may be decreased. Nevertheless, when the affinity separation matrix having a SpG variant or a SpG domain variant of which binding ability to a CH1 region is high as a ligand is used, a high recovery yield may be achieved even in the case where a usage amount of the washing liquid is large. For example, when a chromatography system is used, it can be judged without difficulty whether an impurity can be sufficiently removed or not by monitoring an elution profile.

An impurity is exemplified by an impurity derived from an antibody in addition to a protein and DNA derived from a cultivated host cell. An impurity derived from an antibody is exemplified by a substance derived from an excessively produced antibody and a component generated by misfolding, such as a light chain monomer, a light chain dimer, an antibody aggregate. Since an impurity derived from an antibody has a physical property similar to that of a target antibody fragment, it is generally difficult to separate the impurity. On the one hand, such an impurity derived from an antibody can be efficiently removed by the present invention method. The above-described impurity can be detected by SDS-PAGE, HPLC or the like, but the detection method is not restricted thereto.

### Step 4: Step of separating antibody fragment

In Step 4, the above-described antibody fragment is separated from the affinity separation matrix on which the antibody fragment is adsorbed by using an eluate. By Step 4, the purified antibody fragment can be obtained.

In the present invention, as an eluate to elute the antibody fragment, an acidic aqueous solution can be used. Such an acidic aqueous solution is exemplified by aqueous solutions of acetic acid, citric acid and glycine. When the pH of the aqueous solution is low, the antibody fragment can be efficiently eluted and an amount of the eluate can be reduced. When the pH of the aqueous solution is high, it becomes possible to reduce the damage caused by acid to the antibody fragment. The pH of the eluate is preferably 2.5 or more and 4.0 or less. When the pH is 2.5 or more, the chemical change or the like of the antibody fragment can be suppressed more surely. On the one hand, when the pH is 4.0 or less, the antibody fragment can be eluted more surely. The pH is more preferably 2.8 or more, even more preferably 3.0 or more, and more preferably 3.8 or less, even more preferably 3.5 or less. The purity of the obtained antibody fragment can be determined by SDS-PAGE, HPLC or the like, but the method is not restricted thereto.

### Step 5: Posttreatment step

By the above-described Step 4, an aqueous solution of the antibody fragment is obtained. The antibody fragment may be further purified by salting-out, chromatography, recrystallization or the like, and dried by freeze dry, spray dry, film drying method or the like.

### Step 6: Step of regenerating affinity separation matrix

In Step 6, the affinity separation matrix which is used in the above-described Step 4 and from which the antibody fragment is separated is regenerated by washing with a regenerating solution. It is not needed to necessarily perform Step 6 after the above-described Step 4, and Step 6 may be performed once every three iterations of the above Steps 1 to 3, once every five iterations, or once every ten iterations. Specifically, when a performance of the affinity separation matrix, such as binding capability, is maintained, Step 6 is not necessarily performed. The implementation frequency and condition of Step 6 is also different depending on the liquid sample containing the antibody fragment to be purified.

The "regenerating solution" usable for the regeneration of the affinity separation matrix means an aqueous solution by which purpose such as washing and sterilization can be achieved. More specifically, 1 M acetic acid solution (pH 2.0), 20 mM phosphate - 1% SDS solution (pH 7.0), 6 M guanidine - hydrochloride solution (pH 7.0), 70% ethanol, 0.1 M hydrochloric acid (pH 1.0), 8 M urea solution (pH 10.5), 0.1 M glycine - sodium hydroxide solution (pH 11) can be used as a regenerating solution, but the regenerating solution is not restricted thereto.

The time to treat the affinity separation matrix after the above-described Step 4 by the regenerating solution is not particularly restricted and may be appropriately adjusted, since a damage degree of the Protein G ligand is different depending on the kind of the regenerating solution and the temperature at the treatment. For example, when 0.1 M hydrochloric acid (pH 1.0) is used as a regenerating solution and the temperature during immersion is atmospheric temperature, the time is preferably 1 hour and more preferably 2 hours. When 8 M urea (pH 10.5) is used as a regenerating solution and the temperature during immersion is atmospheric temperature, the time is preferably 1 hour and more preferably 2 hours.

The affinity separation matrix regenerated by Step 6 can be used in the above-described Steps 1 to 3 again.

The present application claims the benefit of the priority date of Japanese patent application No. 2016-148820 filed on July 28, 2016. All of the contents of the Japanese patent application No. 2016-148820 filed on July 28, 2016, are incorporated by reference herein.

### EXAMPLES

Hereinafter, the present invention is described in more detail with Examples; however, the present invention is not restricted to the following Examples.

### Example 1

### (1) Preparation of Fab fragment-containing supernatant

A Fab fragment was designed on the basis of the public information of the sequence of completely humanized anti-TNF α antibody (adalimumab) and selected as an antibody fragment which contained a heavy chain constant region (CH region) but which did not contain an Fc region. A Gene encoding the Fd chain amino acid sequence (CH1 region and VH region, SEQ ID NO: 3) and the light chain amino acid sequence (SEQ ID NO: 4) of the above-described anti-TNF-α antibody was designed and chemically synthesized. The Fab gene was prepared by PCR using the gene as a template. The above-described Fab fragment was produced by using the obtained gene and methylotrophic yeast. The yeast used for producing the Fab fragment was obtained and cultivated in accordance with the method of Examples 1, 8 and 9 of WO 2012/102171. The culture medium containing the produced Fab fragment was centrifuged and the supernatant was recovered. The recovered supernatant was filtrated by using a sterilized filter having a pore diameter of 0.22 µm ("Minisart" manufactured by Sartorius).

### (2) Preparation of Protein G variant carrier

A carrier on which the Protein G domain variant described in WO 2016/031902 was immobilized was prepared. Specifically, a Fab region-binding peptide was immobilized on a cellulose carrier. The Fab region-binding peptide had a 2 domains structure composed of the amino acid sequence of SEQ ID NO: 5 added by the linker sequence between domains and the C-terminal sequence of wild Protein G and Cys at the C-terminal. As the cellulose carrier, crystalline highly-crosslinked cellulose (manufactured by JNC Corporation, corresponding to the gel obtained by the method described in JP 2009-242770 A) was used. The association constants of the above-described Fab region-binding peptide to the Fab of anti-EGFR antibody and anti-TNF α were 10⁶ M⁻¹ or more. The immobilization was performed by similar method to the epoxy-immobilization method described in WO 2016/031902.

### (3) Purification of Fab from Fab fragment-containing supernatant

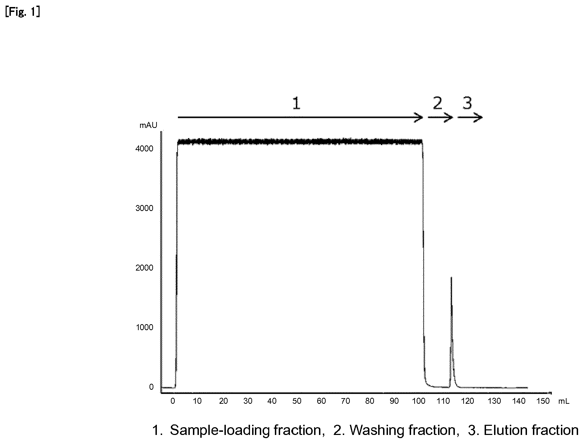

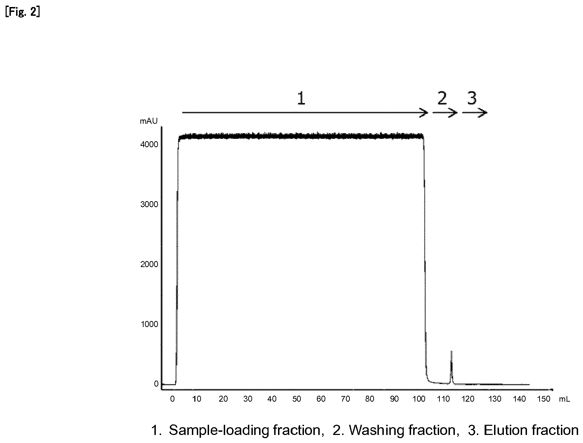

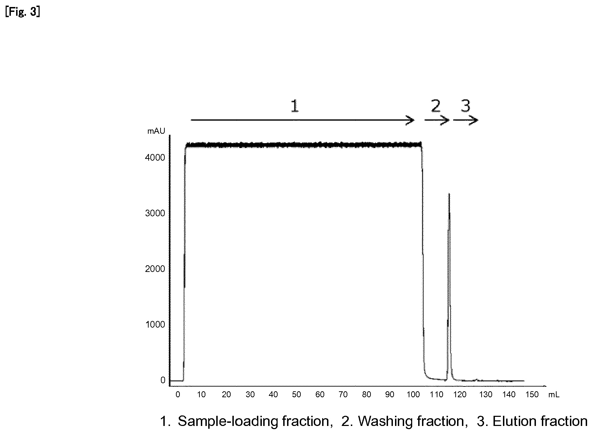

In order to adjust the pH of the Fab fragment-containing supernatant prepared in the above-described (1), the supernatant was diluted 2-fold by using an equilibrating buffer (20 mM NaH₂PO₄ - Na₂HPO₄, 150 mM NaCl, pH 7.4). Then, after the supernatant was filtrated by using a filter having a pore diameter of 0.22 µm ("Minisart" manufactured by Sartorius), the Fab fragment was purified by using a commercially available Protein G carrier ("Protein G Sepharose 4 Fast Flow" manufactured by GE Healthcare) or the Protein G variant carrier prepared in the above-described (2). For comparison, the Fab fragment was also purified by using a commercially available Protein L carrier ("Capto L" manufactured by GE Healthcare) . A commercially available column ("Tricorn 5/50" manufactured by GE Healthcare) was filled with 1 mL-gel of the carriers and connected to chromatography system AKTAavant 25 (manufactured by GE Healthcare). Specifically, the following procedure was performed. First, 5 CV (column volume) of an equilibrating buffer (20 mM Na₂HPO₄ - NaH₂PO₄, 150 mM NaCl, pH 7.4) was flown through the column to equilibrate the carrier. Then, the Fab fragment-containing supernatant prepared in the above-described (1) was diluted 2-fold with the equilibrating buffer to be a sample, and 100 mL of the sample was supplied to the column. Next, after the carrier was washed by flowing 10 CV of the equilibrating buffer, 10 CV of an elution liquid (100 mM glycine - HCl, pH 2.7) was flown to elute the adsorbed Fab fragment. Then, after 3 CV of the equilibrating buffer was flown and 5 CV of 1 M acetic acid aqueous solution was flown to wash the carrier, 5 CV of the equilibrating buffer was flown to complete the purification. The flow rate was adjusted to 1 mL/min in the above procedure. In the any purifications using the carriers, the fractions at the time of the supply of the sample, washing and elution were kept. The obtained elution fraction was neutralized by using 2 M Tris solution. Each chromatogram in the case where a Protein G variant carrier, a commercially available Protein G carrier and a commercially available Protein L carrier were used is shown as Figures 1 to 3. In Figures 1 to 3, each fraction was represented as 1 to 3.

### (4) Identification of component in each fraction

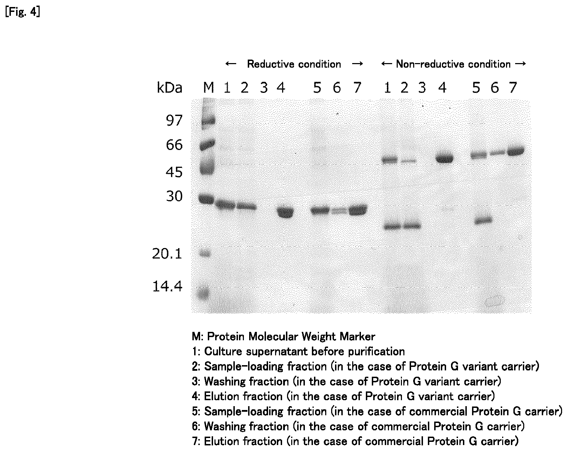

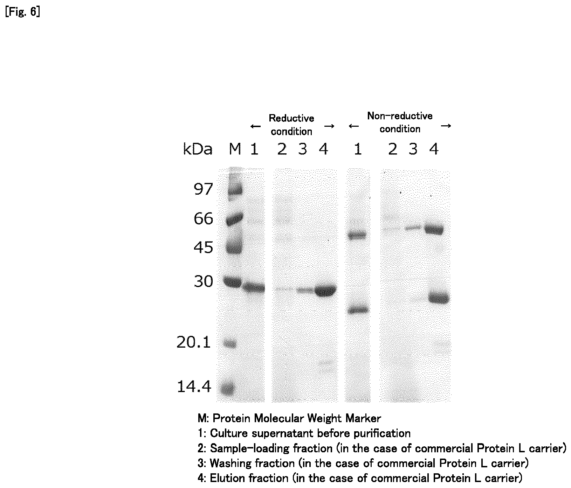

The sample loading fractions, washing fractions and elution fractions obtained by using each carrier in the above-described (3) were analyzed by SDS-PAGE. Specifically, SDS-PAGE was performed by using mini-slab electrophoresis system with a built-in power "PageRun" manufactured by ATTO Corporation and 15% polyacrylamide - precast gel "e·PAGEL" manufactured by ATTO Corporation in accordance with the attached manual. The sample loading fraction was diluted by 10-fold, since the protein concentration was high. The gel was stained and decolorized by using CBB staining solution used for visualization of a protein ("EzStain AQua" manufactured by ATTO Corporation) in accordance with the attached manual. The SDS-PAGE result in the case where a Protein G variant carrier and a commercially available Protein G carrier were used is shown as Figure 4, the enlarged photograph in the case of non-reductive treatment condition is shown as Figure 5, and the SDS-PAGE result in the case where a commercially available Protein L carrier was used is shown as Figure 6.

As the result demonstrated by Figure 4, the band positions of the light chain (molecular weight: 23,412) and the heavy chain (VH region and CH1 region: 23,871) were slightly different but observed around 30 kDa in a reductive condition. In a non-reductive condition, the band of the Fab fragment was observed between 45 kDa and 66 kDa, and a band was also observed between 20.1 kDa and 30 kDa. As the SDS-PAGE result under non-reductive condition demonstrated by Figure 6, when a Protein L carrier was used, the band between 20.1 kDa and 30 kDa was not observed in the sample loading fraction but was observed in the washing fraction. Since a Protein L carrier has a binding ability to a κ light chain, the band can be identified as a component derived from a light chain and is considered to be a light chain monomer from the aspect of the molecular weight. The position of the light chain monomer band in a reductive condition was different from that in a non-reductive condition. Since there is cysteine in the light chain amino acid sequence, the structure of the light chain in a reductive condition is different from that in a non-reductive condition. The difference of the positions is considered to be due to the difference of the structures.

According to Figure 5 which is an expanded figure around 45 to 66 kDa of Figure 4 in a non-reductive condition, there are 2 bands between 45 kDa and 66 kDa in the culture supernatant before the purification. The band having lower molecular weight is considered to be the band of the light chain dimer.

As the result demonstrated in Figure 6, when a Protein L carrier was used, both of the light chain monomer and light chain dimer were mixed in the elution fraction and could not be separated from the Fab fragment.

On the one hand, as Figure 4, when any of a Protein G variant carrier and a commercially available Protein G carrier were used, the band of the light chain monomer existed in the culture supernatant before the purification and the sample loading fraction but could not be observed in the washing fraction and elution fraction. In addition, the band of the light chain dimer was observed in both of the sample loading fractions in the cases of a Protein G variant carrier and a commercially available Protein G carrier but could not observed in the washing fraction and elution fraction. From the results, it was found that an unwanted component and a misfolded protein such as a light chain monomer and a light chain dimer in a culture supernatant can be removed and a Fab fragment can be purified with high purity by using a Protein G carrier.

When a commercially available protein G carrier was used, the fact that there was the band of the Fab in both of the sample loading fraction and washing fraction was different from the case where a protein G variant carrier was used. In other words, the result suggests that a light chain monomer and a light chain dimer can be removed by a commercially available protein G carrier but a Fab fragment was leaked at the time of a sample loading and washing. On the one hand, when a Protein G variant carrier was used, the Fab fragment was not contained in the washing liquid. Thus, the carrier on which a Protein G variant having high association constant to a Fab fragment is immobilized has more excellent ability to maintain a Fab fragment. As a result, a Fab fragment having high purity can be obtained with high recovery yield by the purification step using the carrier.

## Claims

1. A method for producing an antibody fragment,
wherein the antibody fragment comprises a CH1 region and does not comprise an Fc region,
comprising the steps of:
preparing a liquid sample, wherein the liquid sample comprises the antibody fragment and does not comprise an Fc fragment,
contacting the liquid sample with an affinity separation matrix in order to adsorb the antibody fragment on the affinity separation matrix, wherein Protein G, a Protein G domain, a Protein G variant or a Protein G domain variant is immobilized as a ligand on a water-insoluble carrier in the affinity separation matrix,
washing the affinity separation matrix to remove an impurity, and
separating the antibody fragment from the affinity separation matrix.

2. The method according to claim 1, wherein an association constant of the Protein G variant or the Protein G domain variant to the CH1 region is 10⁶ M⁻¹ or more.

3. The method according to claim 2, wherein the Protein G variant or the Protein G domain variant has an amino acid sequence of SEQ ID NO: 5.

4. The method according to any one of claims 1 to 3, wherein the antibody fragment comprises a light chain, and wherein the impurity is 1 or more selected from the group consisting of a light chain monomer, a light chain dimer and an antibody aggregate.

5. The method according to any one of claims 1 to 4, wherein an amount of a washing liquid used for washing the affinity separation matrix is 3 column volume or more.

6. The method according to any one of claims 1 to 5, wherein an eluate used for separating the antibody fragment from the affinity separation matrix is an aqueous solution of 1 or more acids selected from the group consisting of acetic acid, citric acid and glycine.

7. The method according to claim 6, wherein a pH of the aqueous solution is 2.5 or more and 4.0 or less.


SEQUENCE LISTING

<110>  KANEKA CORPORATION

<120>  METHOD FOR PRODUCING ANTIBODY FRAGMENT

<130>  B160710

<150>  JP2016-148820
<151>  2016-07-28

<160>  5

<170>  PatentIn version 3.5

<210>  1
<211>  56
<212>  PRT
<213>  Streptococcus sp. GX7809

<400>  1
Asp Thr Tyr Lys Leu Ile Leu Asn Gly Lys Thr Leu Lys Gly Glu Thr
1               5                   10                  15

Thr Thr Glu Ala Val Asp Ala Ala Thr Ala Glu Lys Val Phe Lys Gln
            20                  25                  30

Tyr Ala Asn Asp Asn Gly Val Asp Gly Glu Trp Thr Tyr Asp Asp Ala
        35                  40                  45

Thr Lys Thr Phe Thr Val Thr Glu
    50                  55

<210>  2
<211>  56
<212>  PRT
<213>  Streptococcus sp. GX7809

<400>  2
Thr Thr Tyr Lys Leu Val Ile Asn Gly Lys Thr Leu Lys Gly Glu Thr
1               5                   10                  15

Thr Thr Lys Ala Val Asp Ala Glu Thr Ala Glu Lys Ala Phe Lys Gln
            20                  25                  30

Tyr Ala Asn Asp Asn Gly Val Asp Gly Val Trp Thr Tyr Asp Asp Ala
        35                  40                  45

Thr Lys Thr Phe Thr Val Thr Glu
    50                  55

<210>  3
<211>  224
<212>  PRT
<213>  Homo sapiens

<400>  3
Glu Val Gln Leu Val Glu Ser Gly Gly Gly Leu Val Gln Pro Gly Arg
1               5                   10                  15

Ser Leu Arg Leu Ser Cys Ala Ala Ser Gly Phe Thr Phe Asp Asp Tyr
            20                  25                  30

Ala Met His Trp Val Arg Gln Ala Pro Gly Lys Gly Leu Glu Trp Val
        35                  40                  45

Ser Ala Ile Thr Trp Asn Ser Gly His Ile Asp Tyr Ala Asp Ser Val
    50                  55                  60

Glu Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Lys Val Ser Tyr Leu Ser Thr Ala Ser Ser Leu Asp Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

<210>  4
<211>  214
<212>  PRT
<213>  Homo sapiens

<400>  4
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Arg Asn Tyr
            20                  25                  30

Leu Ala Trp Tyr Gln Gln Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Arg Tyr Asn Arg Ala Pro Tyr
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  5
<211>  56
<212>  PRT
<213>  Artificial Sequence
<220>
<223>  SpG domain mutant

<400>  5
Thr Thr Tyr Lys Leu Ile Leu Asn Gly Lys Thr Leu Thr Gly Tyr Thr
1               5                   10                  15

Thr Ala Ile Ala Ala Asp Ala Ala Thr Ala Glu Ile Val Leu Lys Gln
            20                  25                  30

Phe Ala Asn Asp Asn Gly Ile Asp Gly Glu Trp Thr Tyr Asp Asp Ala
        35                  40                  45

Thr Lys Thr Phe Thr Val Thr Glu
    50                  55